# EUROPEAN PATENT SPECIFICATION

(11) **EP 1 298 069 B1**
(45) Date of publication and mention of the grant of the patent: **06.05.2009**
(21) Application number: 02256712.7
(22) Date of filing: 26.09.2002
(51) Int. Cl.: B65D 75/26, B65D 75/58, G01N 33/53, G01N 33/92, G01N 33/66, C12Q 1/24

(54) **Device for use in monitoring swab technique**
Vorrichtung zur Überprüfung von Techniken, die Tupfer beinhalten
Dispositif pour le contrôl d'échantillonneurs de surface

(30) Priority: 26.09.2001 GB 0123151
(43) Date of publication of application: 02.04.2003
(73) Proprietor: 3M Innovative Properties Company, St. Paul MN 55133-3427 (US)
(72) Inventor: Ramsay, Catherine Mary, Cowbridge, Vale of Glamorgan CF71 7ER (GB); Simpson, William John, Lingfield, Surrey RH7 6HJ (GB)
(74) Representative: Thomas, Simon

(56) References cited:
- EP-A- 0 171 150
- EP-A- 0 915 336
- EP-A- 0 916 405
- WO-A-00/54029
- WO-A-93/19363
- WO-A-97/06436
- WO-A-98/48280
- WO-A-98/50778
- WO-A-98/56568
- WO-A-99/33714
- US-A- 4 963 325

## Description

The present invention is concerned with a device suitable for use in determining the accuracy of a swab technique.

Various tests are available that can be used to assess the cleanliness of a surface. Such tests include those based on the detection of ATP using the firefly luciferase reaction, tests based on the detection of protein using colorimetry, tests based on the detection of micro-organisms using microbiological culture techniques, and tests based on detection of micro-organisms using immunochemical techniques. Surfaces can be sampled using either a swab device, or by direct contact with an agar medium.

Although the above tests are useful in the detection of a contaminated surface, they are typically prone to errors. These errors may be due to the performance of the measurement apparatus (a light detection device, such as a luminometer, colour detection by human assessor, or test result interpretation by human assessor).

It is also believed that errors may be due to variations in the assay conditions, for example being caused by assay temperature. Errors may also be due to the detection "reagents" used in the test systems, including firefly luciferase reagents for ATP, colorimetric reagents for protein detection, microbiological media for bacteriological analysis etc. For example, abused or badly stored reagents can give rise to false-positive or false-negative results.

Errors may also arise due to differences in sampling procedure. For example, "swabs" are often used for sample collection in such hygiene assays. These can give rise to variation due to the presence or absence of extraction agents and/or surface active components.

Furthermore, errors can arise due to differences in operator technique during the performance of the assay itself. Although this may be due to an error on the part of the operator, it is more common that the variance in performance is a result of subtle differences in technique.

All of the above sources of potential error ultimately lead to a reduction in confidence in the validity of such test results. In the case of safety critical applications there are significant consequences if a set of invalid results are generated.

There are a number of different methods for dealing with the inherent variability of surface hygiene tests. Such methods include:
1. Use of chemical standards (such as the use of solutions of ATP to calibrate ATP tests)(Jago, PH, Stanfield, G, Simpson, WJ & Hammond, JRM 1989. In ATP Bioluminescence: Rapid Methods in Microbiology, Society of Applied Microbiology Technical Series, Vol 26, Stanley PE et al [eds] pp 53-61). These can be applied in two ways. In the external calibration technique, the test response is compared to that obtained in the presence of known concentrations of the analyte. In the internal calibration technique, a known amount of analyte is added to the test after measurement of the sample signal. The ratio of the signal from the test sample to that of the standard can be used to calculate the amount of analyte present in the sample.
2. Use of a light sensitive derivative of the analyte to assure test performance. This is the basis of the PhotoQuant® technique for assay calibration (Method for calibrating chemical assays, PCT/GB95/00794);
3. Use of low level light emitting devices to assure the performance of instrumentation, or use of coloured cards to act as an aid to the judgement (by eye) of colorimetric tests etc. For example, radioactive material employing a scintillant are employed in the Biolink light standards (Leaback, DH, Easy-to-use light standards as aids to luminometry, Szalay, AA et al [Eds], pp 33-37, Bioluminescence of the VII International Symposium on Bioluminescence and Chemiluminescence, John Wiley & Son, Chichester 1993).

Whilst these methods are useful in some circumstances, there are a number of limitations. All of the approaches identified above are limited by the fact that they do not respond to differences in operator swab technique. Faulty sampling techniques (which may include failure to swab the required area, or failure to apply sufficient pressure during the swabbing process) can lead to low results (or even negative results).

It is therefore an aim of the present invention to alleviate at least some of the disadvantages highlighted above.

It is also an aim of the present invention to provide a standard surface for use in the monitoring of swab technique in an analytical method.

It is a further aim of the present invention to provide a method of monitoring swab technique.

Therefore, according to a first aspect of the present invention, there is provided a device according to claim 1. The device is adapted for use in monitoring a swab method with the device includes a first substrate substantially adjacent a second substrate, the first substrate and the second substrate having disposed therebetween a test material with the first and second substrates being sealed together substantially around their periphery.

Advantageously, the test material includes a predetermined amount of test analyte. The analyte may include ATP, a protein, other chemical materials (such as adenosine diphosphate (ADP), adenosine monophosphate (AMP) pyrophosphate (PPi), guanosine triphosphate (GTP), guanosine diphosphate (GDP), guanosine monophosphate (GMP), cytidine triphosphate (CTP), cytidine diphosphate (CDP), cytidine monophosphate (CMP), deoxyribonucleic acid (DNA), ribonucleic acid (RNA) various minerals (including Ca, Zn, Mg, Mn and Co), sugars (including lactose, glucose and maltose), lipids and fatty acids, microbial cell wall and cell membrane materials (such as peptidoglycan, techoic acid and lipopolysaccharides), enzymes (such as proteases, adenylate kinase, invertase, melibiase, and alkaline phosphatase) and/or a microorganism.

Advantageously, the test material is protected from the environment prior to use, thereby substantially reducing (or preferably substantially inhibiting) contamination of the test material by the external environment. The test material is typically disposed between the first substrate and the second substrate under aseptic conditions.

The first substrate and the second substrate are sealed together substantially at their periphery so as to form a pouch or sachet, the test material being substantially contained in the pouch. The first substrate and the second substrate are sealed together by use of an hermetic bond, or the like. The bond may be formed by use of an adhesive, such as a polyurethane adhesive, alternatively, they be may joined together by use of heat sealing the first substrate and/or the second substrate or by a pressure sensitive adhesive. Heat sealing may include the use of heat sealing of a plastic polymer surface. It is also envisaged that the first substrate and the second substrate may be coextrusion laminated; the first substrate and/or the second substrate preferably being formed from materials such as ethylene vinyl acetate, ethylene methacylate or ethylene vinyl alcohol.

The first substrate and the second substrate may be of metal, including aluminium (such as aluminium foil), or a plastics material.

Each substrate may be of the same material. It is therefore envisaged that the first substrate and the second substrate are formed from the same sheet of material. In this embodiment, the sheet may be folded about a fold-line, the fold-line forming a sealed edge of the pouch. However, it is preferred that each substrate is of a different material.

The first substrate and the second substrate have respective internal surfaces which have different wetting properties.

The first substrate has a surface which is hydrophobic and the second substrate has a surface which is hydrophillic. Each substrate may be treated with, for example a polymer so as to form the hydrophillic and/or hydrophobic surface. However, it is also envisaged that each substrate may be of a hydrophillic or hydrophobic material. Advantageously, the test material disposed between the first substrate and the second substrate will preferentially wet the hydrophillic surface leaving the hydrophobic surface substantially unwetted.

This feature is particularly advantageous as, although in principle an equal volume of material should adhere to each substrate, in practice the surface is inconsistently wetted resulting in an unknown (or uneven) amount of analyte remaining on each substrate. Therefore, the use of different surfaces, one being hydrophillic and the other being hydrophobic, results in substantially all of the analyte being on the hydrophillic surface.

In this particular embodiment, it is preferred that the test material includes a hyrophillic surface enhancer, such as a detergent, which increases the probability of the test material wetting the hydrophillic surface as opposed to wetting the hydrophobic surface. Preferred hydrophillic surface enhancers include benzalkonium chloride, benzethonium chloride and chlorhexidine gluconate.

It is further desirable that the device (and therefore the test material) is stable prior to being used in the monitoring of, for example, swab technique. It is therefore preferred that the test material includes a stabilising agent.

The stabilising agent may include a chelating agent when the test material includes ATP (the non-enzymic breakdown of ATP is inhibited by chelating divalent cations). A preferred chelating agent includes ethylene diamine tetraacetic acid (EDTA). In this particular embodiment, it is preferred that the test material has a low pH value (for example less than 7.0) so as to restrict base-catalysed hydrolysis of ATP. In addition, it is preferred that the test material includes ADP when ATP is present in the test material (ADP has the advantage of reducing the hydrolysis of ATP to ADP).

It is preferred that when the test material includes a protein, the stabilising agent includes a compound which reduces water availability (for example glycerol) therefore improving protein stability.

Preferably, when the test material includes a microorganism the stabilising agent includes a quaternary ammonium detergent or biguanide, such as a benzethonium chloride or chlorhexidine gluconate, which advantageously acts as an inhibitor of enzyme action, and as a preservative against microbiological degradation.

A particularly preferred test material for use in the present invention, when the present invention is suitable for use in monitoring the swab method in an ATP assay and protein-based hygiene tests, includes a blend comprising:

| | |
|---|---|
| Glycerol | 50g |
| Chlorhexidine gluconate | 2g |
| Bovine serum albumin | 0.5g |
| ATP | 0.18x10⁻⁹g |
| De-ionized water | 50g |

According to a further aspect of the present invention, there is provided a method of manufacturing a device as described in claim 1, for use in monitoring a swab technique, the method including providing a first substrate, applying a test material to a portion of the first substrate, covering at least the test material on the first substrate with a second substrate, and joining the second substrate to the first substrate so as to encapsulate the test material between the first substrate and the second substrate.

The device manufactured according to this aspect of the present invention is substantially as described herein before with reference to the first aspect of the present invention.

It is, however, envisaged that the device according to the first aspect of the present invention may be manufactured by methods known in the art of making sachets and/or pouches. Such methods include those disclosed in (but not limited to) US 5879769, US 5445821, US 4747782, EP 1013193 and WO 9856568.

It is preferred that the test material is applied to the first substrate as a (relatively) dry, localised spot, (which is particularly preferred) or as a homogeneously applied film.

Typically, the test material has a thickness on the first substrate less than about 1mm, preferably less than about 0.3mm.

It is preferred that the first substrate includes a first release portion and the second substrate includes a second release portion, each release portion being arranged about a peripheral edge of the respective substrate. The first release portion being preferably substantially not connected to the second release portion.

According to yet a further aspect of the present invention, there is provided a method of monitoring a swab technique using a device according to claim 1. Basically the method includes:
a) providing a device according to claim 1 comprising a first substrate substantially adjacent a second substrate, the first substrate and the second substrate having disposed therebetween a test material including a predetermined amount of an analyte;
b) peeling the first substrate and second substrate away from each other, at least particularly along their periphery, where sealed together to reveal the test material;
c) swabbing the test material with a swab; and
   monitoring the amount of analyte present on the swab.

The device is substantially as described herein before.

The test material is typically disposed between the first substrate and the second substrate under aseptic conditions.

The amount of analyte present on the swab may be monitored by methods known in the art. The mode of monitoring will, of course, be dependent on the analyte contained in the test material. The analyte may be monitored using, for example, apparatus currently sold by Biotrace Limited under the Trade Marks CLEAN-TRACE and PROTECT. Although it is envisaged that any suitable apparatus known to a person skilled in the art could be used.

The amount of analyte monitored on the swab is compared to the amount of analyte present in the test material. The comparison provides an indication of how accurate the swab technique is. For example if monitoring the amount of analyte on the swab identifies only about 50% of analyte present in the test material has been detected, the swab method may be considered as being unacceptable.

The present invention will now be described with reference to the accompanying Figures, which are given by way of example only, wherein:

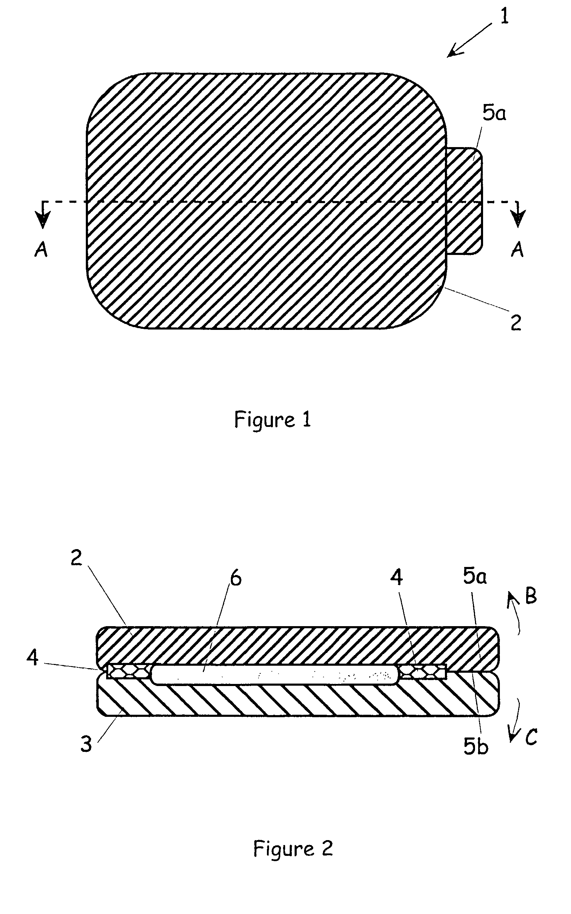

FIGURE 1 represents a plan view of a device according to the present invention; and
FIGURE 2 represents a cross-sectional view along the line A-A of Figure 1.

Referring to the figures, where like numerals have been used to represent like parts, there is provided a test sachet generally indicated by the numeral 1. A first aluminium substrate 2 is adhered about the periphery to a second plastics substrate 3 by use of an adhesive 4. A portion of aluminium substrate 2 is not adhered to plastics substrate 3 so as to provide release tabs 5a and 5b. Test material 6 containing a predetermined amount of analyte is disposed between aluminium substrate 2 and plastics substrate 3.

In use, release tab 5a is pulled in the direction of arrow B and release tab 5b is pulled in the direction of arrow C, thereby separating the substrates 2 and 3 and permitting access to test material 6.

A swab (not shown) is then wiped over surface 6. The amount of analyte present on the swab is then monitored using apparatus known to a person skilled in the art. The result obtained is then compared to the predetermined amount of analyte present on the first substrate so as to determine the accuracy of the swab technique.

## Claims

1. A device adapted for use in monitoring a swab method, the device including a first substrate substantially adjacent a second substrate, the first substrate and the second substrate having disposed therebetween a test material, **characterized in that** the first substrate and the second substrate are sealed together by use of an hermetic bond substantially at their periphery, so as to form a pouch or sachet, the test material being substantially contained in the pouch and the first and second substrates in use are separable to allow access to the test material when the test is to be carried out, and wherein the first substrate and the second substrate have respective internal surfaces which have different wetting properties, the first substrate having a surface which is hydrophobic and the second substrate having a surface which is hydrophilic.

2. A device according to claim 1, wherein the test material includes a predetermined amount of test analyte.

3. A device according to claim 2, wherein the test analyte includes any one or more of ATP, a protein, other chemical materials (such as adenosine diphosphate (ADP), adenosine monophosphate (AMP), pyrophosphate (PPi), guanosine triphosphate (GTP), guanosine diphosphate (GDP), guanosine monophosphate (GMP), cytidine triphosphate (CTP), cytidine diphosphate (CDP), cytidine monophosphate (CMP), deoxyribonucleic acid (DNA), ribonucleic acid (RNA), various minerals (including Ca, Zn, Mg, Mn and Co), sugars (including lactose, glucose and maltose), lipids and fatty acids, microbial cell wall and cell membrane materials (such as peptidoglycan, techoic acid and lipopolysaccharides), enzymes (such as proteases, adenylate kinase, invertase, melibiase, and alkaline phosphatase) and/or a microorganism.

4. A device according to any preceding claim, wherein test material is protected from the environment prior to use, thereby substantially reducing (or preferably substantially inhibiting) contamination of the test material by the external environment.

5. A device according to claim 1, wherein the bond is formed by use of an adhesive (such as a polyurethane adhesive), or by means of heat sealing the first substrate and/or the second substrate to form a hermetic bond, or by use of a pressure sensitive adhesive.

6. A device according to any preceding claim wherein the first substrate and the second substrate are coextrusion laminated.

7. A device according to claim 6, wherein the first substrate and/or the second substrate are formed from ethylene vinyl acetate, ethylene methacrylate or ethylene vinyl alcohol.

8. A device according to any of claims 1 to 5, wherein the first substrate and the second substrate are manufactured from a metal, including aluminium (such as aluminium foil), or a plastics material.

9. A device according to any preceding claim, wherein the first substrate and the second substrate are formed from the same sheet of material, preferably the sheet may be folded about a fold-line, the fold-line forming a sealed edge of the pouch.

10. A device according to claim 1, wherein the test material disposed between the first substrate and the second substrate will preferentially wet the hydrophilic surface leaving the hydrophobic surface substantially unwetted.

11. A device according to claim 10, wherein the test material includes a hydrophilic surface enhancer, such as a detergent, which increases the probability of the test material wetting the hydrophilic surface as opposed to wetting the hydrophobic surface, preferably the hydrophilic surface enhancers include benzalkonium chloride, benzethonium chloride and chlorhexidine gluconate.

12. A device according to any preceding claim, wherein the test material includes a stabilising agent, such as a chelating agent when the test material includes ATP.

13. A device according to claim 12, wherein the stabilising agent includes a compound which reduces water availability (for example glycerol) therefore improving protein stability when the test material includes a protein.

14. A device according to claim 12, wherein the stabilising agent includes a quaternary ammonium detergent or biguanide, such as a benzethonium chloride or chlorhexidine gluconate when the test material includes a micro-organism

15. A device according to any preceding claim, suitable for use in monitoring the Swab method in an ATP assay and protein-based hygiene test, wherein the test material includes a blend comprising:
| | |
|---|---|
| Glycerol | 50 g |
| Chlorhexidine gluconate | 2 g |
| Bovine serum albumin | 0.5 g |
| ATP | 0.18 x 10⁻⁹g |
| De-ionized water | 50 g |

16. A device according to claim 15, wherein the test material has a thickness on the first substrate less than about 1 mm, preferably less than about 0.3 mm.

17. A device according to any preceding claim, wherein the first substrate includes a first release portion and the second substrate includes a second release portion, each release portion being arranged about a peripheral edge of the respective substrate, preferably the first release portion being substantially not connected to the second release portion.

18. A method of manufacturing a device, adapted for monitoring a swab technique according to claim 1, the method including providing a first substrate, applying a test material to a portion of the first substrate, covering at least the test material on the first substrate with a second substrate, and joining the second substrate to the first substrate substantially at their periphery so as to encapsulate the test material between the first substrate and the second substrate.

19. A method according to claim 18, wherein the test material is applied to the first substrate as a dry, localised spot, or as a homogeneously applied film.

20. A method of monitoring a swab technique, which method includes:
(a) providing a device according to claim 1, comprising a first substrate substantially adjacent a second substrate, the first substrate and the second substrate having disposed there between a test material including a predetermined amount of an analyte;
(b) peeling the first substrate and second substrate away from each other, at least particularly along their periphery, where sealed together to reveal the test material;
(c) swabbing the test material with a swab; and monitoring the amount of analyte present on the swab.

21. A method according to claim 20, wherein the test material is disposed between the first substrate and the second substrate under aseptic conditions.

## Patentansprüche

1. Vorrichtung, die zur Verwendung beim Überwachen eines Abstrichverfahrens angepasst ist, wobei die Vorrichtung ein erstes Substrat einschließt, das im Wesentlichen unmittelbar an ein zweites Substrat angrenzt, wobei das erste Substrat und das zweite Substrat dort dazwischen ein Testmaterial angeordnet aufweisen, **dadurch gekennzeichnet, dass** das erste Substrat und das zweite Substrat mithilfe einer hermetischen Verbindung, im Wesentlichen an ihrer Peripherie, dergestalt zusammengesiegelt sind, um einen Beutel oder ein Sachet zu bilden, wobei das Testmaterial im Wesentlichen im Beutel enthalten ist und die ersten und zweiten Substrate bei Gebrauch trennbar sind, um den Zugang zum Testmaterial zu erlauben, wenn der Test durchgeführt werden soll, und worin das erste Substrat bzw. das zweite Substrat entsprechende interne Oberflächen aufweisen, welche unterschiedliche Benetzungseigenschaften aufweisen, wobei das erste Substrat eine Oberfläche aufweist, die hydrophob ist und das zweite Substrat eine Oberfläche aufweist, die hydrophil ist.

2. Vorrichtung nach Anspruch 1, worin das Testmaterial eine vorbestimmte Menge des Testanalyts einschließt.

3. Vorrichtung nach Anspruch 2, worin der Testanalyt jedwedes eine oder mehr von Folgendem einschließt: ATP, ein Protein, andere chemische Materialien (wie zum Beispiel Adenosindiphosphat (ADP), Adenosinmonophosphat (AMP), Pyrophosphat (PPi), Guanosintriphosphat (GTP), Guanosindiphosphat (GDP), Guanosinmonophosphat (GMP), Cytidintriphosphat (CTP), Cytidindiphosphat (CDP), Cytidinmonophosphat (CMP), Desoxyribonukleinsäure (DNA), Ribonukleinsäure (RNA), verschiedene Mineralien (einschließlich Ca, Zn, Mg, Mn und Co), Zucker (einschließlich Lactose, Glucose und Maltose), Lipide und Fettsäuren, mikrobielle Zellwand- und Zellmembranmaterialien (wie zum Beispiel Peptidoglykan, Teichonsäure und Lipopolysaccharide), Enzyme (wie zum Beispiel Proteasen, Adenylatkinase, Invertase, Melibiase und alkalische Phosphatase) und/oder einen Mikroorganismus.

4. Vorrichtung nach einem der vorangegangenen Ansprüche, worin das Testmaterial vor Gebrauch vor der Umgebung geschützt ist, wodurch die Kontamination des Testmaterials durch die externe Umgebung im Wesentlichen reduziert (oder bevorzugt im Wesentlichen inhibiert) wird.

5. Vorrichtung nach Anspruch 1, worin die Bindung durch Verwendung eines Klebers (wie zum Beispiel eines Polyurethan-Klebers) oder mittels Hitzesiegelung des ersten Substrats und/oder des zweiten Substrats zum Bilden einer hermetischen Verbindung oder durch Verwendung eines druckempfindlichen Klebers gebildet ist.

6. Vorrichtung nach einem der vorangegangenen Ansprüche, worin das erste Substrat und das zweite Substrat durch Coextrusion laminiert sind.

7. Vorrichtung nach Anspruch 6, worin das erste Substrat und/oder das zweite Substrat aus Ethylen-Vinylacetat, Ethylen-Methacrylat oder Ethylen-Vinylalkohol gebildet sind.

8. Vorrichtung nach einem der Ansprüche 1 bis 5, worin das erste Substrat und das zweite Substrat aus einem Metall, einschließlich Aluminium (wie zum Beispiel Aluminiumfolie) oder einem Kunststoffmaterial hergestellt sind.

9. Vorrichtung nach einem der vorangegangenen Ansprüche, worin das erste Substrat und das zweite Substrat aus dem gleichen Folienmaterial gebildet sind, wobei die Folie bevorzugt über eine Faltlinie gefaltet sein kann, wobei die Faltlinie eine versiegelte Kante des Beutels bildet.

10. Vorrichtung nach Anspruch 1, worin das zwischen dem ersten Substrat und dem zweiten Substrat angeordnete Testmaterial bevorzugt die hydrophile Oberfläche benetzt, wobei es die hydrophobe Oberfläche im Wesentlichen unbenetzt zurücklässt.

11. Vorrichtung nach Anspruch 10, worin das Testmaterial einen hydrophilen Oberflächenverbesserer, wie zum Beispiel ein Detergens, einschließt, welcher die Wahrscheinlichkeit erhöht, dass das Testmaterial eher die hydrophile Oberfläche als die hydrophobe Oberfläche benetzt, wobei bevorzugt die hydrophilen Oberflächenverbesserer Benzalkoniumchlorid, Benzethoniumchlorid und Chlorhexidinglukonat einschließen.

12. Vorrichtung nach einem der vorangegangenen Ansprüche, worin das Testmaterial einen Stabilisator, wie zum Beispiel einen Chelatbildner einschließt, wenn das Testmaterial ATP einschließt.

13. Vorrichtung nach Anspruch 12, worin der Stabilisator eine Verbindung einschließt, welche die Wasserverfügbarkeit (zum Beispiel Glycerol) reduziert, wobei er folglich die Proteinstabilität verbessert, wenn das Testmaterial Protein einschließt.

14. Vorrichtung nach Anspruch 12, worin der Stabilisator ein quartäres Ammoniumdetergens oder Biguanid, wie zum Beispiel ein Benzethoniumchlorid oder Chlorhexidinglukonat einschließt, wenn das Testmaterial einen Mikroorganismus einschließt.

15. Vorrichtung nach einem der vorangegangenen Ansprüche, die zur Verwendung bei der Überwachung des Abstrichverfahrens in einem ATP-Assay und einem auf Protein basierenden Hygienetest geeignet ist, worin das Testmaterial eine Mischung einschließt, die Folgendes umfasst:
| | |
|---|---|
| Glycerol | 50 g |
| Chlorhexidinglukonat | 2 g |
| Rinderserumalbumin | 0,5 g |
| ATP | 0,18 x 10⁻⁹ g |
| Entionisiertes Wasser | 50 g |

16. Vorrichtung nach Anspruch 15, worin das Testmaterial auf dem ersten Substrat eine Dicke von weniger als ca. 1 mm, bevorzugt weniger als ca. 0,3 mm aufweist.

17. Vorrichtung nach einem der vorangegangenen Ansprüche, worin das erste Substrat ein erstes Freigabeteil einschließt und das zweite Substrat ein zweites Freigabeteil einschließt, wobei jedes Freigabeteil um eine periphere Kante des entsprechenden Substrats angeordnet ist, wobei das erste Freigabeteil im Wesentlichen nicht mit dem zweiten Freigabeteil verbunden ist.

18. Verfahren zur Herstellung einer Vorrichtung, die zur Überwachung eines Abstrichverfahrens nach Anspruch 1 angepasst ist, wobei das Verfahren die Bereitstellung eines ersten Substrats einschließt, Auftragen eines Testmaterials auf ein Teil des ersten Substrats, Abdecken von mindestens dem Testmaterial auf dem ersten Substrat mit einem zweiten Substrat und miteinander Verbinden des zweiten Substrats mit dem ersten Substrat im Wesentlichen an ihrer Peripherie dergestalt, um das Testmaterial zwischen dem ersten Substrat und dem zweiten Substrat einzukapseln.

19. Verfahren nach Anspruch 18, worin das Testmaterial auf das erste Substrat als ein trockener, lokalisierter Fleck oder als ein homogen aufgebrachter Film aufgetragen wird.

20. Verfahren zum Überwachen eines Abstrichverfahrens, welches Verfahren Folgendes einschließt:
(a) Bereitstellen einer Vorrichtung nach Anspruch 1, umfassend ein erstes Substrat im Wesentlichen unmittelbar benachbart zu einem zweiten Substrat, wobei das erste Substrat und das zweite Substrat dort dazwischen ein Testmaterial, einschließlich einer vorbestimmten Menge eines Analyts angeordnet aufweisen;
(b) Abziehen des ersten Substrats und zweiten Substrats voneinander, mindestens insbesondere ihrer Peripherie entlang, an der sie zusammengesiegelt sind, um das Testmaterial freizulegen;
(c) Anfertigen eines Abstrichs vom Testmaterial mit einem Abstrichtupfer und Überwachen der auf dem Abstrich vorhandenen Analytmenge.

21. Verfahren nach Anspruch 20, worin das Testmaterial unter aseptischen Bedingungen zwischen dem ersten Substrat und dem zweiten Substrat angeordnet ist.

## Revendications

1. Dispositif adapté pour être utilisé dans le contrôle d'une méthode de frottis, le dispositif comprenant un premier substrat en grande partie adjacent à un second substrat, le premier substrat et le second substrat ayant disposée entre ceux-ci une substance de test, **caractérisé en ce que** le premier substrat et le second substrat sont scellés ensemble à l'aide d'une liaison hermétique en grande partie à leur périphérie, de façon à former une poche ou un sachet, la substance de test étant en grande partie contenue dans la poche et les premier et second substrats à l'utilisation étant séparables pour permettre l'accès à la substance de test lorsque le test doit être effectué, et dans lequel le premier substrat et le second substrat ont des surfaces internes respectives qui ont différentes propriétés de mouillage, le premier substrat ayant une surface qui est hydrophobe et le second substrat ayant une surface qui est hydrophile.

2. Dispositif selon la revendication 1, dans lequel la substance de test comprend une quantité prédéterminée d'analyte de test.

3. Dispositif selon la revendication 2, dans lequel l'analyte de test comprend l'un quelconque ou plusieurs parmi l'ATP, une protéine, d'autres substances chimiques (telles que l'adénosine diphosphate (ADP), l'adénosine monophosphate (AMP), le pyrophosphate (PPi), la guanosine triphosphate (GTP), la guanosine diphosphate (GDP), la guanosine monophosphate (GMP), la cytidine triphosphate (CTP), la cytidine diphosphate (CDP), la cytidine monophosphate (CMP), un acide désoxyribonucléique (ADN), un acide ribonucléique (ARN), divers minéraux (dont Ca, Zn, Mg, Mn et Co), des sucres (dont le lactose, le glucose et le maltose), des lipides et acides gras, des matières de paroi cellulaire et de membrane cellulaire microbiennes (telles que le peptidoglycane, l'acide techoïque et des lipopolysaccharides), des enzymes (telles que des protéases, l'adénylate kinase, l'invertase, la mélibiase et la phosphatase alcaline) et/ou un microorganisme.

4. Dispositif selon l'une quelconque des revendications précédentes, dans lequel la substance de test est protégée de l'environnement avant utilisation, réduisant par là en grande partie (ou de préférence inhibant en grande partie) la contamination de la substance de test par l'environnement extérieur.

5. Dispositif selon la revendication 1, dans lequel la liaison est formée en utilisant un adhésif (tel qu'un adhésif en polyuréthane), ou au moyen du scellage à chaud du premier substrat et/ou du second substrat pour former une liaison hermétique, ou en utilisant un adhésif sensible à la pression.

6. Dispositif selon l'une quelconque des revendications précédentes dans lequel le premier substrat et le second substrat sont stratifiés par coextrusion.

7. Dispositif selon la revendication 6, dans lequel le premier substrat et/ou le second substrat sont formés à partir d'éthylène-acétate de vinyle, d'éthylène-méthacrylate ou d'éthylène-alcool vinylique.

8. Dispositif selon l'une quelconque des revendications 1 à 5, dans lequel le premier substrat et le second substrat sont fabriqués à partir d'un métal, dont l'aluminium (tel qu'une feuille d'aluminium), ou d'une matière plastique.

9. Dispositif selon l'une quelconque des revendications précédentes, dans lequel le premier substrat et le second substrat sont formés à partir de la même feuille de matière, de préférence la feuille peut être pliée le long d'une ligne de pliage, la ligne de pliage formant un bord scellé de la poche.

10. Dispositif selon la revendication 1, dans lequel la substance de test disposée entre le premier substrat et le second substrat mouillera préférentiellement la surface hydrophile laissant la surface hydrophobe en grande partie non mouillée.

11. Dispositif selon la revendication 10, dans lequel la substance de test comprend un améliorant de surface hydrophile, tel qu'un détergent, qui augmente la probabilité du mouillage de la surface hydrophile par la substance de test par opposition au mouillage de la surface hydrophobe, de préférence les améliorants de surface hydrophiles comprennent le chlorure de benzalkonium, le chlorure de benzéthonium et le gluconate de chlorhexidine.

12. Dispositif selon l'une quelconque des revendications précédentes, dans lequel la substance de test comprend un agent stabilisant, tel qu'un agent chélatant lorsque la substance de test comprend de l'ATP.

13. Dispositif selon la revendication 12, dans lequel l'agent stabilisant comprend un composé qui réduit la disponibilité de l'eau (par exemple le glycérol) améliorant par conséquent la stabilité des protéines lorsque la substance de test comprend une protéine.

14. Dispositif selon la revendication 12, dans lequel l'agent stabilisant comprend un détergent ammonium quaternaire ou un biguanide, tel que le chlorure de benzéthonium ou le gluconate de chlorhexidine lorsque la substance de test comprend un microorganisme.

15. Dispositif selon l'une quelconque des revendications précédentes, convenant pour être utilisé dans le contrôle de la méthode de frottis dans un dosage d'ATP et un test d'hygiène à base de protéine, dans lequel la substance de test comprend un mélange comprenant :
| | |
|---|---|
| Glycérol | 50 g |
| Gluconate de chlorhexidine | 2 g |
| Albumine de sérum bovin | 0,5 g |
| ATP | 0,18 x 10⁻⁹ g |
| Eau désionisée | 50 g. |

16. Dispositif selon la revendication 15, dans lequel la substance de test a une épaisseur sur le premier substrat inférieure à environ 1 mm, de préférence inférieure à environ 0,3 mm.

17. Dispositif selon l'une quelconque des revendications précédentes, dans lequel le premier substrat comprend une première partie de séparation et le second substrat comprend une seconde partie de séparation, chaque partie de séparation étant disposée au voisinage d'un bord périphérique du substrat respectif, de préférence la première partie de séparation étant en grande partie non reliée à la seconde partie de séparation.

18. Procédé de fabrication d'un dispositif, adapté pour contrôler une technique de frottis selon la revendication 1, le procédé comprenant de fournir un premier substrat, appliquer une substance de test sur une partie du premier substrat, recouvrir au moins la substance de test sur le premier substrat avec un second substrat et unir le second substrat au premier substrat en grande partie à leur périphérie de façon à encapsuler la substance de test entre le premier substrat et le second substrat.

19. Procédé selon la revendication 18, dans lequel la substance de test est appliquée sur le premier substrat sous forme d'un dépôt ponctuel localisé et sec, ou sous forme d'un film appliqué de façon homogène.

20. Procédé de contrôle d'une technique de frottis, lequel procédé comprend de :
(a) fournir un dispositif selon la revendication 1, comprenant un premier substrat en grande partie adjacent à un second substrat, le premier substrat et le second substrat ayant disposée entre ceux-ci une substance de test comprenant une quantité prédéterminée d'un analyte ;
(b) décoller le premier substrat et le second substrat l'un de l'autre, au moins en particulier le long de leur périphérie, où ils étaient scellés ensemble pour exposer la substance de test ;
(c) prélever la substance de test avec un tampon ; et contrôler la quantité d'analyte présente sur le tampon.

21. Procédé selon la revendication 20, dans lequel la substance de test est disposée entre le premier substrat et le second substrat dans des conditions aseptiques.
